(19) **Europäisches Patentamt / European Patent Office / Office européen des brevets**

(11) **EP 3 459 082 B1**

(12) **EUROPEAN PATENT SPECIFICATION**

| | |
|---|---|
| (45) Date of publication and mention of the grant of the patent: **27.05.2020 Bulletin 2020/22** | (51) Int Cl.: **G16H 50/50** (2018.01)   **A61B 8/15** (2006.01) |
| (21) Application number: **17822595.9** | (86) International application number: **PCT/EP2017/082233** |
| (22) Date of filing: **11.12.2017** | (87) International publication number: **WO 2018/108820 (21.06.2018 Gazette 2018/25)** |

(54) **METHOD OF, AND APPARATUS FOR, NON-INVASIVE MEDICAL IMAGING USING WAVEFORM INVERSION**

VERFAHREN UND VORRICHTUNG ZUR NICHT-INVASIVEN MEDIZINISCHEN BILDGEBUNG MITTELS WELLENFORMINVERSION

PROCÉDÉ ET APPAREIL D'IMAGERIE MÉDICALE NON EFFRACTIVE UTILISANT UNE INVERSION DE FORME D'ONDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2016 GB 201621436**

(43) Date of publication of application:
**27.03.2019 Bulletin 2019/13**

(73) Proprietor: **Calderon Agudo, Oscar**
**London SW11 2EX (GB)**

(72) Inventor: **Calderon Agudo, Oscar**
**London SW11 2EX (GB)**

(74) Representative: **Beck Greener LLP**
**Fulwood House**
**12 Fulwood Place**
**London WC1V 6HR (GB)**

(56) References cited:
**WO-A2-2010/042146     US-A1- 2012 283 566**

- LI CUIPING ET AL: "Toward a practical ultrasound waveform tomography algorithm for improving breast imaging", PROGRESS IN BIOMEDICAL OPTICS AND IMAGING, SPIE - INTERNATIONAL SOCIETY FOR OPTICAL ENGINEERING, BELLINGHAM, WA, US, vol. 9040, 20 March 2014 (2014-03-20), pages 90401P-90401P, XP060036483, ISSN: 1605-7422, DOI: 10.1117/12.2043686 ISBN: 978-1-5106-0027-0
- OSCAR CALDERON AGUDO ET AL: "3D imaging of the breast using full-waveform inversion", PROCEEDINGS ON THE INTERNATIONAL WORKSHOP ON MEDICAL ULTRASOUND TOMOGRAPHY, 1.-3. NOV. 2017, SPEYDER, GERMANY, KARLSRUHE, BADEN : KIT SCIENTIFIC PUBLISHING, 2018, DE, 1 November 2017 (2017-11-01), pages 99-110, XP009504285, ISBN: 978-3-7315-0689-8

**Description**

The present invention relates to an improved method of, and apparatus for, non-invasive imaging of regions of the body using ultrasound-generated data. More particularly, the present invention relates to a method for non-invasive imaging of bone- or gas-containing regions of the body using waveform inversion of ultrasound generated data, which may be useful in the diagnosis and prognosis of pathologies, particularly brain pathologies such as stroke.

Medical ultrasound is a well established technology that uses high frequency acoustic waves (generally at frequencies in excess of 20 kHz) to generate acoustic images of the human or animal body. The application of this technique ranges from obstetric ultrasound to identification of pathologies, echocardiography or to drive interventions in real time.

Different types of images can be generated using ultrasound technology. 2D acoustic impedance maps can be generated. Alternatively, blood flow maps or motion of tissue over time can be measured and recorded using Doppler effects.

The advantages of ultrasound are many - in particular, this technique provides a real time, portable and cheap imaging technology which does not require ionising radiation. In the majority of medical applications, reflected acoustic energy is detected to generate acoustic impedance maps. Reflection mode imaging such as this is very successful in particular areas of medical imaging - for example, the imaging of a foetus in the womb, or the detection of breast cancer. The relative simplicity of such imaging enables images to be provided in real-time because the algorithms that generate such images are only based on a delay and stack strategy. The only information used to create the image is the time at which the echoes arrive at the receiver, and the strength of those echoes.

However, such techniques have met with limited success when attempting to image regions of the body comprising bone or gas pockets. This is because such materials within the body cause scattering, attenuation and phase distortion of ultrasound signals, making imaging difficult. In addition, imaging of the brain is particularly difficult due to the presence of a strong ultrasound reflector (the skull) in close proximity to the ultrasound source.

For example, "Ultrasound reflection mode computed tomography through a skullbone", J Ylitalo, IEEE transactions on biomedical engineering Vol 37, No. 11 (November 1990) discloses an ultrasonic reflection mode CT method which showed some success in respect of paediatric brain diagnosis however was unreliable when applied to thicker adult skulls.

An alternative technique to reflection mode analysis is transmission mode analysis. "Computerized ultrasound tomography of the human head: Experimental results", K.A. Dines et al, Ultrasonic Imaging, 3, 342-351 (1981) discloses methods for using transmission analysis human skulls. However, again, whilst encouraging results were seen for paediatric skulls, the technique proved unreliable when applied to adult skulls.

A further alternative ultrasound technique is to utilise dispersion to categorise brain injuries. US 8,834,376 discloses methods for utilising dispersion to provide information on the composition of inter-cranial tissues. However, such a method cannot provide imaging of brain regions.

US 2012/283566 discloses a system for imaging soft tissue which includes ultrasound emitters and receivers, and a processor configured for determining an observed differential time-of-flight (ToF) dataset and generating an acoustic speed rendering of the tissue based on the observed differential ToF dataset.

To date, the only successful techniques for imaging the brain using ultrasound have relied upon obtaining imaging through thinner regions of the skull, either using naturally-thinner skulls (e.g. a young child's skull) or through natural or man-made (e.g. bored) acoustic windows within the skull. The same applies to other areas of the body containing significant sources of acoustic absorption, reflection or dispersion, or heterogenous regions (e.g. bone or gas regions or interfaces therebetween). Therefore, there exists a technical problem in the art that ultrasound imaging of such regions cannot reliably be performed using known techniques.

According to a first aspect of the present invention there is provided a non-invasive method of generating image data of intra-cranial tissue using ultrasound energy that is transmitted across a head of a subject through the skull of the subject, the method comprising the steps of: a) providing an ultrasound observed data set derived from a measurement of one or more ultrasound waveforms generated by at least one source of ultrasound energy, the ultrasound energy being detected by a plurality of receivers located at an opposing side of a region within the intra-cranial cavity with respect to at least one source such that the receivers detect ultrasound waveforms from the source which have been transmitted through the skull and intra-cranial cavity, the observed data set comprising a plurality of observed data values; b) providing at least one starting model for at least a portion of the head comprising a skull component and a soft tissue component, the skull component comprising a plurality of model parameters representative of the physical properties and morphology of the skull through which intra-cranial tissue is being imaged, and the soft tissue component comprising a plurality of parameters representative of the physical properties of the intra-cranial tissue being imaged; c) generating a predicted data set comprising a plurality of predicted data values from the starting model of the skull and of the intra-cranial tissue; d) comparing the observed and predicted data values in order to generate an updated model of at least one physical property within at least a region of the intra-cranial cavity; and e) using the updated model to image a region of the inter-cranial cavity to identify tissue composition and/or morphology within the intra-cranial cavity.

According to a second aspect of the present invention, there is provided a non-invasive method of generating image data of a body part of a subject using ultrasound energy that is transmitted through the body part of the subject, the body part containing at least one interface between bone, soft tissue and/or gas, the method comprising the steps of: a) providing an ultrasound observed data set derived from a measurement of one or more ultrasound waveforms generated by at least one source of ultrasound energy, the ultrasound energy being detected by a plurality of receivers located at an opposing side of a region within the body part with respect to at least one source such that the receivers detect ultrasound waveforms from the source which have been transmitted through the body part, the observed data set comprising a plurality of observed data values; b) providing at least one starting model representative of the body part being imaged, the starting model comprising first and second components, the first component comprising a plurality of model parameters representative of the physical properties and morphology of the bone and/or gas within the body part of the subject to be imaged and having at least one modelled region having an acoustic velocity below 700 m/s and/or above 2300 m/s and the second component comprising a plurality of parameters representative of the physical properties of the soft tissue within the body part of the subject to be imaged; c) generating a predicted data set comprising a plurality of predicted data values from the starting model; d) comparing the observed and predicted data values in order to generate an updated model of at least one physical property within at least a region of the body part; and e) using the updated model to image a region of the body part to identify tissue composition and/or morphology within the body part.

Further embodiments of the first and second aspects are specified in dependent claims 2-5 and 7-12.

According to a third aspect of the present invention, there is provided a computer system comprising a processing device configured to perform the method of the first or second aspects.

According to a fourth aspect of the present invention, there is provided a computer readable medium comprising instructions configured when executed to perform the method of the first or second aspects.

According to a fifth aspect of the present invention, there is provided a computer system comprising: a processing device, a storage device and a computer readable medium of the third aspect.

Embodiments of the present invention will now be described in detail with reference to the accompanying drawings, in which:

Figure 1 shows a plan view of a measurement data acquisition apparatus exemplified by use on the head of a subject;

Figure 2 shows a side view of the acquisition apparatus of Figure 1;

Figure 3 shows a target model of a region of an inter-cranial cavity to be imaged;

Figure 4 shows the target model with only the skull shown for clarity;

Figure 5 shows trace data generated from the target model of Figures 3 and 4;

Figure 6 shows a starting model for use with the target model test of Figures 3 and 4;

Figure 7 shows an image generated using the method of the present invention to recover the target model of Figures 3 and 4; and

Figure 8 shows a flow chart of an embodiment of the invention.

The present invention, in embodiments, relates to a novel method for imaging of body structures using ultrasound. In embodiments of the present invention, both transmitted and reflected energy is recorded. Using both these techniques, it is possible to obtain information relating to tissues and body structures that lie behind bone (and/or gas) and generate images of such.

The method of the present invention uses Full Waveform Inversion (FWI) or a variant thereof.

Whilst FWI has limitations with regard to providing real-time images, developments in the implementation and processing of the images should bring down the turn-around time by at least an order of magnitude in due course. This is offset by the potential advantages of the high resolution of the images generated by FWI, which may potentially rival MRI.

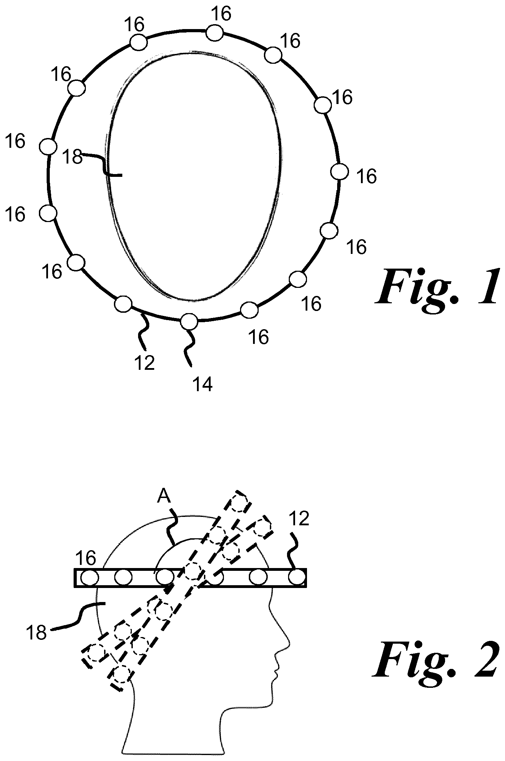

Figures 1 and 2 shows an exemplary experimental configuration 10 for obtaining imaging information relating to the head. However, it is to be understood that the configuration described herein could be used on other parts of the body containing regions of bone and/or gas, for example, the leg, arm, chest or other region of interest. In addition, the measurement is not limited to humans and other animals may comprise subjects to be measured and imaged. It is also to be understood that the general experimental configuration as shown in Figures 1 and 2 is not to be taken as limiting.

Any configuration for data acquisition which can generate a suitable measured data set for subsequent analysis could be used with the present invention.

The experimental configuration 10 comprises a ring 12 which includes at least one ultrasound source 14 and a plurality of receivers 16. The ring 12 is arranged around a head 18 of a subject. The source 14 and/or receivers 16 are acoustically coupled to the head 18. This may be achieved by either locating the source 14 and/or receivers 16 directly on the surface of the head. Alternatively, the head 18 may be immersed in a suitable acoustic medium (such as water) to enable the required coupling.

As shown in Figure 2, the ring 12 may be oriented at any suitable angle A with respect to the head for data acquisition. Data may be acquired at multiple different angles with respect to the head. In preferred arrangements, these different angles are all selected such that the resulting imaging planes intersect. This may enable construction of three dimensional images of a particular region where the planes intersect. However, this is not to be taken as limiting and data may be acquired in two or three dimensions and in any planar configuration.

For example, in one embodiment, the ring 12 may translate in one or more directions to obtain data in a number of parallel, offset planes to capture "slices" through the head.

In addition, whilst a ring 12 is shown in Figures 1 and 2, this need not be the case and a helmet or other wearable device may be provided. Multiple rings may be provided within the wearable device to capture simultaneously or sequentially multiple slices through the head.

The source 14 generates acoustic ultrasound waves having sufficient vibrational energy to penetrate the skull of the subject and generate sufficient return signals to aid useful detection. The source 14 may be any suitable ultrasound generator. The skilled person will be readily aware of the type of generators that are suitable for use with the acquisition approach of the present invention.

The source 14 is, in embodiments, a point source or a close approximation of a point source. In other words, the source 14 is a source of ultrasonic waves which emits in all directions equally. In the context of the experimental configuration 10, this may be understood to be an isotropic source for the purpose of the detectors 16 such that the source 14 is an isotropic radiator in directions of interest for the measurement (e.g. within the ring 12). It is not essential or necessary that the source 14 is isotropic, or even generates a signal at all, in directions which do not intersect the target structure (e.g. directions away from the head).

However, whilst point sources (or functionally similar sources) are desirable, this need not be the case. Instead, the source 14 may have some directionality within the target region. Whilst it is generally preferred to use isotropic or quasi-isotropic emitters, there may be situations where more focused beams (for example, in particular planes or at particular distances) may be useful. However, in contrast to known ultrasound imaging arrangements, the distortion of signals is a useful parameter which contains information that can be recovered and used to provide imaging data. In contrast, known imaging arrangements are configured to avoid distortion due to the difficulty in correcting for such effects.

As shown in Figures 1 and 2, a plurality of receivers 16 is provided. The receivers 16 may comprise any suitable ultrasonic detection apparatus. The receivers 16 are connected to a trace acquisition apparatus such as a computer or other electronic storage device.

Whilst a single source 14 and multiple receivers 16 are shown in Figures 1 and 2, it is to be understood that multiple sources 14 may also be used at different locations. For example, each source 14 may also comprise a receiver 16 and vice versa. Each of these sources could be used to build up multiple traces from different source locations. For example, in the exemplary model described below, a total of 450 source/receiver units are used. As each source emits, the other 449 receivers detect the reflected and/or transmitted waves. Each source 14 is then used in turn to generate signals that can be detected, resulting in a total of 450 different ultrasonic traces. These traces may then be used together as an observed data set for analysis.

In use, ultrasonic waves generated by the source 14 propagate into the head 18 of the subject. The waves are transmitted and refracted through the layers of bone and/or brain matter and/or reflected off the interfaces between them and/or scattered from other heterogeneities within the head and a plurality of return signals is detected by the detectors 16.

An observed data set comprises a measurement, by the multiplicity of receivers 16, of transmitted, reflected and/or refracted acoustic waves originating from the source 14. In general, a partial reflection of the acoustic wave occurs at a boundary or interface between two dissimilar materials, or when the elastic properties of a material changes. Each detected return signal forming an ultrasound trace has an approximate travel time from the source 14 which, for reflected waves, is a two-way travel time from the source 14 to the reflecting element (for example, the interior surface of the skull opposite from the source 14) and back to the respective detector 16.

In the experimental configuration 10 shown in Figures 1 and 2, the source 14 is located on one side of the head under investigation. The plurality of receivers 16 on the other side record the transmitted signal, whilst the receivers 16 near the source 14 record the reflected ultrasound waves.

The time-variation of the reflected and transmitted waves (i.e. the waveforms detected by the receivers 16) is recorded during a pre-determined time period. This time period is selected to be sufficient to capture both reflected and transmitted arrivals that contain information of the properties of the head. A typical value may be of the order of 300 - 500 μs.

The source 14 may emit ultrasonic waves at any desired frequency, plurality of discrete frequencies or continuous band of frequencies (e.g. a broadband signal). In general, a multiplicity of frequencies is used to provide greater resolution and detail in the produced images. Whilst high frequencies provide greater resolution of smaller physical features, the penetration depth of lower frequencies is better for imaging further through the skull. The present invention is operable to use a range of frequencies which can be resolved together or individually as required.

In embodiments, the frequencies span a range from 400 kHz to 1.3 MHz. However, other ranges could be used with the present invention, and a continuous bandwidth and/or discrete frequency selections ranging from approximately 50 kHz up to 5MHz could be used with the present invention.

Some or all of the multiple sources 14 (if present) may be activated simultaneously to generate a single large source gather. Alternatively, the sources 14 may be actuated individually.

An observed ultrasonic data set is then acquired by recording the waveforms at the receivers 16 after emission by the one or more sources 14. The observed data set may comprise a plurality of waveform traces. For example, there will be a single waveform trace for each source/receiver combination. These traces form the observed ultrasonic data set.

A predicted data set is obtained by modelling data from a starting model of the acoustic properties of the area investigated, such as wave speed velocity. The waveforms in the observed data set are then analysed by comparing them to the waveforms in the predicted data set in order to recover a model of at least one acoustic property of the body. This can be done using the full-waveform inversion (FWI) method by minimising the least-squares norm between observed and predicted data.

Key to this process of modelling and imaging a region of a subject is the ultrasonic velocity $V_p$. In a portion of the volume of a subject, $V_p$ may be estimated in various ways.

The observed ultrasonic data set is then used as part of a waveform inversion process to extract acoustic properties of the tissues forming the patient's head. An example of full waveform inversion (FWI) will now be described.

FWI is a known method for analysing data, particularly in the field of seismology. FWI is able to produce models of physical properties such as $V_p$ in the measured region that have high fidelity and that are well resolved spatially. FWI seeks to extract the acoustic properties of the imaged region of the head from the recorded observed data set. A detailed velocity estimate can be produced using an accurate model with variations on the scale of the ultrasonic wavelength.

The FWI technique involves generating a two or three dimensional model to represent the measured portion of the subject's head or body region and attempting to modify the properties and parameters of the model to generate predicted data that matches the experimentally obtained ultrasonic trace data.

The predicted data is calculated from the model typically using the full two-way wave equation. FWI is an iterative process requiring a starting model. A sufficiently accurate starting model for FWI may be provided by travel-time tomography.

FWI can extract many physical properties ($V_p$ and shear-wave velocities, attenuation, density, anisotropy) of the modelled portion of the subject's body. However, $V_p$, the P-wave velocity, is a particularly important parameter which the subsequent construction of the other parameters depends heavily upon. Nevertheless, other parameters may be used with the present invention, either alone or in combination. Attenuation and density may also be important parameters in the context of medical imaging. The nature and number of parameters used in a model of a portion of the subject's body will be readily apparent to the skilled person.

The FWI technique seeks to obtain an accurate and high resolution model of the measured region of the subject's body which generates predicted data that matches the recorded data. As set out above, determination of $V_p$ is a focus of the technique. However, other parameters such as density and attenuation may also be modelled. Predicted data is calculated using the full two-way wave equation. This is known as the forward problem. This equation can be in the time domain, the frequency domain, or other suitable domains, and it may be elastic or acoustic, isotropic or anisotropic, and may include other physical effects such as attenuation and dispersion. In most cases FWI proceeds using the acoustic approximation with a single component modelled wavefield.

An example of the FWI process in accordance with an embodiment of the present invention will now be described. To test the applicability of the method of the present invention, a target model was developed. The target model is representative of a model of a human head and is used as a target for the FWI process. In other words, the target model is used to generate an example synthetic observed data set, and from this, a starting model is iteratively modified using the FWI process to arrive at a final model. The final model is then compared to the target model to validate the process.

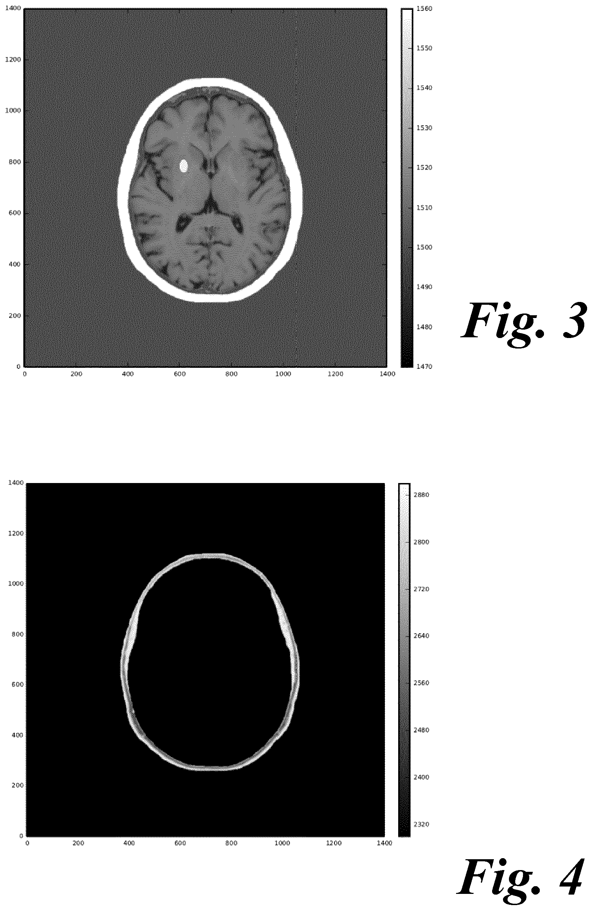

The target model is a 2D synthetic model of a human head and is shown in Figures 3 and 4. In order to build the model, a Magnetic Resonance Imaging (MRI) image was used in combination with tables of known acoustic velocities of various brain and skull tissues.

Note that the skull has velocities beyond the range of the scale of Figure 3 and it appears to be constant velocity. However, within the skull, there are also variations of velocity as it can be seen in Figure 4 which shows a different scale of velocities to illustrate better the variations in acoustic velocity. A blood clot is shown as a white ellipse around coordinates (800,600) in Figure 3, and this is part of the target model to be resolved.

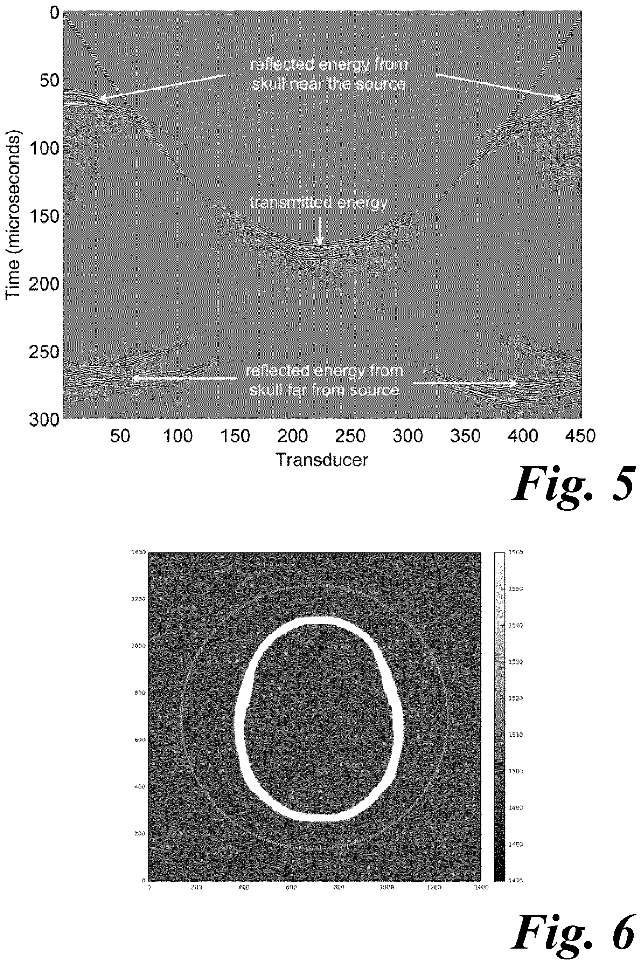

A single source gather from a synthetic observed data set generated from the target model of Figures 3 and 4 can be seen in Figure 5. The substantially sinusoidal pattern in Figure 5 results from the arrangement of receivers in a circle around the target. The gather of Figure 5 is generated by applying the isotropic acoustic wave equation to the model of Figures 3 and 4 and then modelling the reflected and refracted signals as they would be detected. The modelled source gather is made up of individual traces at receiver position showing pressure recorded as a function of time.

In general, the parameters of the model are estimated at a plurality of points set out in a grid or volume, but they may be estimated from any suitable parameterisation. The model is used to generate a modelled representation of the ultrasound data set, known as the predicted data set. The predicted data set is then compared to the real-world experimentally obtained observed data set. Then, through use of numerical iteration, the parameters of the model are modified until the predicted data set generated from the model matches the actual observed data to a sufficient degree of accuracy or until sufficient convergence is obtained. This will be explained below.

A general method to update a model will now be described. FWI typically operates on the principle of iteratively updating the starting model to minimise or maximise an objective function through repeated steepest-descent direction calculation, or an analogous technique. An objective function represents some measure of the mismatch or some measure of similarity between the recorded data and the predicted data. A measure of mismatch, obtained for example by subtracting two traces, should be minimised; whereas a measure of similarity, obtained for example by cross-correlating two traces, should be maximised.

Due to the non-linearity in the relationship between the model and the data, the objective function used in FWI will oscillate with changes in the model. This makes it necessary to have a sufficiently accurate starting model for global minimum convergence. The objective function can be formulated in the frequency domain, the time domain or other suitable domain. The choice of domain allows the use of preconditioning on either the data or the model update direction that could improve convergence or reduce the non-linearity of the inverse problem.

Frequency domain inversion is equivalent to time domain inversion if all the frequencies are inverted simultaneously. However, the global minimum broadens at lower frequencies reducing how accurate the starting model needs to be for local optimisation method to be successful.

A starting model requires at least two components: (1) A component of the model that represents regions, for example bone or gas, that have values of Vp that differ substantially from values that are typical of soft tissue within the body, and (2) a component of the model that represents values typical of soft tissue within the body. The component of the body represented by component (1) will typically have values of Vp that lie below 700 m/s or above 2300 m/s whereas the portion of the body represented by component (2) will typically have values of Vp that lie close to 1500 m/s. With regard to soft tissue, the acoustic velocity varies from around 1450 m/s for fat to about 1730 m/s for skin. Obtaining a satisfactory starting model for component (2) will normally be straightforward whereas obtaining a satisfactory starting model for component (1) will normally be both essential and more difficult.

An example of a basic starting model for the head of a subject is shown in Figure 6. Figure 6 shows a substantially horizontal section through the model showing the skull and source/receiver locations (the outer circle).

The starting model makes no assumptions in relation to the brain's velocities and it is a simple homogeneous model of 1500 m/s. The true velocities for the skull are used in the starting model in this example. The skull corresponds to starting model component (1) and the brain to starting model component (2).

A total of 450 transducers equally spaced around the skull are used and they can act as sources or receivers. Since each source activates one of the transducers while the remaining 449 act as receivers, and all transducers are used as sources resulting in a total of 450 independent experiments.

Commonly, localised gradient-based methods are used with FWI. These methods iteratively update an existing model in a direction that derives from the objective function's direction of steepest descent.

There are numerous ways to quantify the difference (also known as the residual) between the data sets. However, amongst the most common is a least-squares formulation where the sum of the squares of the differences between the two data sets is minimised over all sources and receivers and over all times. In other words, a model is sought that minimises the $L_2$-*norm* of the data residuals.

The $L_2$-*norm* expresses the misfit between the two data sets as a single number. This parameter is known as the objective function, although it often takes other names such as the misfit function, the cost function or the functional. The objective function $f$ is a real, positive, scalar quantity, and it is a function of the model $m$.

In practice, a factor of a half is often included in the definition of the objective function $f$ because it makes later results simpler. The objective function $f(m)$ is shown in equation 1):

$$1) \qquad f(\mathbf{m}) = \tfrac{1}{2}||\delta\mathbf{d}||^2 = \tfrac{1}{2}\delta\mathbf{d}^\dagger\delta\mathbf{d} = \tfrac{1}{2}\sum^{n_s}\sum^{n_r}\sum^{n_t}|d_{pred} - d_{obs}|^2$$

where $n_s$, $n_r$ and $n_t$ are the number of sources, receivers and time samples in the data set.

In the time-domain the data and the data residuals will be real quantities. However, in the frequency domain the data will in general be complex, as will the source and sometimes the model properties. Equation 1) is correctly recited for complex data. However, in the frequency domain, $n_t$ would be expressed as $n_f$, i.e. a summation over frequency rather than time.

FWI is a local iterative inversion scheme. FWI comprises numerous different methods. The method described herein is one possible implementation of an FWI method suitable for use with the present invention. However, the skilled person would readily be aware of alternative methods that could be used with the present invention.

A starting model $\mathbf{m_0}$ that is assumed to be sufficiently close to the true, ideal model is prepared. The process then makes a series of step-wise improvements to this model which successively reduces the objective function towards zero. Therefore, across an iterative step of the calculation, the objective function needs to be considered for a starting model $\mathbf{m_0}$ and a new model $\mathbf{m = m_0 + \delta m}$.

For a scalar function of a single scalar variable, the Taylor series can be used, truncated to second order. This generates equation 2):

$$2) \quad f(\mathbf{m}) = f(\mathbf{m_0} + \delta\mathbf{m}) = f(\mathbf{m_0}) + \delta\mathbf{m}^T \frac{\partial f}{\partial \mathbf{m}}\bigg|_{\mathbf{m}=\mathbf{m_0}} + \tfrac{1}{2}\delta\mathbf{m}^T \frac{\partial^2 f}{\partial \mathbf{m}^2}\bigg|_{\mathbf{m}=\mathbf{m_0}} \delta\mathbf{m} + \mathcal{O}(\delta\mathbf{m}^3)$$

Differentiating this express with respect to m, and setting the result to zero to minimise $f$ with respect to $\mathbf{m = m_0 + \delta m}$, equation 2) becomes:

$$3) \quad \frac{\partial f}{\partial \mathbf{m}}\bigg|_{\mathbf{m}=\mathbf{m_0}+\delta\mathbf{m}} = 0 + \frac{\partial f}{\partial \mathbf{m}}\bigg|_{\mathbf{m}=\mathbf{m_0}} + \frac{\partial^2 f}{\partial \mathbf{m}^2}\bigg|_{\mathbf{m}=\mathbf{m_0}} \delta\mathbf{m} + \mathcal{O}(\delta\mathbf{m}^2) = 0$$

Then, neglecting second order terms, equation 4) can be derived which expresses the updated to the model $\delta\mathbf{m}$:

$$4) \quad \delta\mathbf{m} \approx - \left(\frac{\partial^2 f}{\partial \mathbf{m}^2}\right)^{-1} \frac{\partial f}{\partial \mathbf{m}} \equiv -\mathbf{H}^{-1}\nabla_{\mathbf{m}}f$$

Where $\nabla_{\mathbf{m}}f$ is the gradient of the objective function $f$ with respect to the model parameters, and $\mathbf{H}$ is the Hessian matrix of second differentials, both evaluated at $\mathbf{m_0}$.

If the model has $M$ parameters, then the gradient is a column vector of length $M$ and the Hessian is an $M$ x $M$ symmetric matrix.

If the number of model parameters $M$ is large, then calculating the Hessian is computationally demanding, and inverting the Hessian exactly is normally computationally intractable. Consequently the method that is typically used is to replace the inverse of the Hessian in equation (9) by a simple scalar $\alpha$ (referred to as the step length). Equation 4) can then be expressed as:

$$5) \quad \delta\mathbf{m} = -\alpha \frac{\partial f}{\partial \mathbf{m}} = -\alpha \nabla_{\mathbf{m}}f$$

Based on equation 5), conventional FWI can use the method of steepest descent. This involves essentially 5 steps:

1. Start from model $\mathbf{m_0}$;
2. Evaluate the gradient $\nabla_{\mathbf{m}}f$ of the objective function for the current model;
3. Find the step length $\alpha$;
4. Subtract $\alpha$ times the gradient from the current model to obtain a new model; and
5. Iterate from step 2 using the new model until the objective function is minimised.

To calculate the gradient and determine the step length, a Jacobian matrix is used as set out in equation 6):

$$6) \qquad \nabla_{\mathrm{m}} f = \frac{\partial f}{\partial \mathbf{m}} = \frac{\partial}{\partial \mathbf{m}} \left( \frac{1}{2} \delta \mathbf{d}^T \delta \mathbf{d} \right) = \left( \frac{\partial \mathbf{d}}{\partial \mathbf{m}} \right)^T \delta \mathbf{d} = \mathbf{J}^T \delta \mathbf{d}$$

where $\mathbf{J}$ is the Jacobian matrix.

A wave equation for a predicted data set $\mathbf{d}$ generated by a source $\mathbf{s}$ can be written as:

$$7) \qquad \qquad \mathbf{Ap = s}$$

Where the data set $\mathbf{d}$ is a subset of the full wavefield $\mathbf{p}$ extracted using the diagonal matrix $\mathbf{D}$ that has non-zero unit values only where there are observed data. That is, as set out in equation 8);

$$8) \qquad \qquad \mathbf{d = Dp}$$

Equation 7) can then be differentiated with respect to $\mathbf{m}$, which is equal to zero because $\mathbf{s}$ and $\mathbf{m}$ are independent:

$$9) \qquad \frac{\partial \mathbf{A}}{\partial \mathbf{m}} \mathbf{p} + \mathbf{A} \frac{\partial \mathbf{p}}{\partial \mathbf{m}} = \frac{\partial \mathbf{s}}{\partial \mathbf{m}} = 0$$

Equation 9) is then pre-multiplied by the matrix $\mathbf{D}$ to extract the wavefield only at the points where data exists. The Jacobian can then be rewritten as:

$$10) \qquad \mathbf{J} = \frac{\partial \mathbf{d}}{\partial \mathbf{m}} = \mathbf{D} \frac{\partial \mathbf{p}}{\partial \mathbf{m}} = -\mathbf{D} \mathbf{A}^{-1} \frac{\partial \mathbf{A}}{\partial \mathbf{m}} \mathbf{p}$$

From equation 10), an expression for the gradient can be derived by recognising that $\mathbf{D}^T \delta \mathbf{d} = \delta \mathbf{d}$ and substituting equation 10) into equation 6), to derive an expression for the gradient:

$$11) \qquad \nabla_{\mathrm{m}} f = -\mathbf{p}^T \left( \frac{\partial \mathbf{A}}{\partial \mathbf{m}} \right)^T \left( \mathbf{A}^{-1} \right)^T \delta \mathbf{d}$$

So to find the gradient, the forward wavefield p is calculated, the numerical operator $\mathbf{A}$ is differentiated with respect to the model parameters and the final term of equation 11) is calculated, which represents a back-propagated residual wavefield.

These terms are then multiplied together for all times and all sources, and summed together to give a value corresponding to each parameter within the model, typically to give one value of the gradient at each grid point within the model.

The final term in equation 11) can be written to arrive at equation 12):

$$12) \qquad \qquad \mathbf{A}^T \delta \mathbf{p} = \delta \mathbf{d}$$

Equation 12) simply describes a wavefield $p$ that is generated by a (virtual) source $\delta \mathbf{d}$, and that is propagated by the operator $\mathbf{A}^T$ which is the adjoint of the operator in the original wave equation. So the term that we need to compute in equation 11) is just the solution of a modified wave equation with the data residuals used as a source.

It is then necessary to compute the step length $\alpha$. Starting from a current model $\mathbf{m_0}$ that generates data $\mathbf{d_0}$ and residuals $\delta \mathbf{d_0}$, a new model $\mathbf{m_1} = \mathbf{m_0} + \delta \mathbf{m_1}$ that generates data $\mathbf{d_1}$ and residuals $\delta \mathbf{d_1}$, where $\delta \mathbf{m}$ is a small change in the opposite direction to the gradient.

Therefore, the aim is to find a new model $\mathbf{m_\alpha} = \mathbf{m_0} + \alpha \, \delta \mathbf{m}$ that generates residuals $\delta \mathbf{m_\alpha}$, where $\alpha$ minimises:

$$13) \qquad \frac{1}{2}\left\|\delta\mathbf{d}_\alpha\right\|^2$$

Assuming a linear relationship:

$$14) \qquad \delta\mathbf{d}_\alpha = \delta\mathbf{d}_0 + \alpha(\mathbf{d}_1 - \mathbf{d}_0) = \delta\mathbf{d}_0 + \alpha(\delta\mathbf{d}_1 - \delta\mathbf{d}_0)$$

By rearranging and differentiating with respect to $\alpha$, setting the differential equal to zero and solving for $\alpha$, equations 15) and 16) can be derived:

$$15) \qquad \alpha = \frac{\delta\mathbf{d}_0^T\mathbf{q}}{\mathbf{q}^T\mathbf{q}}$$

$$16) \qquad \mathbf{q} = \delta\mathbf{d}_0 - \delta\mathbf{d}_1$$

So, to calculate the step length, a forward calculation is made with a perturbed model, and the residuals from both the original and the perturbed models combined to form equation 15).

Once $\alpha$ has been found, the original starting model $\mathbf{m}_0$ can be replaced by $\mathbf{m}_\alpha$ and the step of the iterative calculation is complete. This process can then be repeated.

Note that iteration is necessary because the problem to be solved is non-linear and the inverse problem has been linearised in particular stages. Implicitly, the method invokes the Born approximation. The Born approximation assumes that the perturbation to a wavefield produced by changing a model is linearly related to the change in the model. This is equivalent to considering only first-order scattering by the perturbation.

Ideally, the above method will lead to a convergence to a model which is a correct representation of the skull of the subject under investigation. However, there are some difficulties associated with obtaining correct convergence.

As set out above, FWI methodology for the objective function above relies upon a gradient decent method to solve the inverse problem. This requires that the starting model should match the observed travel times to within half a cycle. However, real ultrasonic data are limited in their frequency bandwidth. This means that real ultrasonic signals are oscillatory.

An inaccurate starting model may predict data that are more than half a cycle in error with respect to the observed data. Such a situation is described as "cycle skipped". When this occurs, because the methodology seeks only a local minimum, FWI will tend to modify the model such that the predicted and observed data are brought into alignment at the nearest cycle, and this will neither correspond to their correct alignment nor to the correct model. This misalignment to the nearest cycle will reduce the data misfit, and typical FWI schemes will become stranded in this position - they will have become stuck in a local minimum in the data misfit function rather than being able to find the global minimum which corresponds to the true model.

Other variations of the functional can be combined with the method of the present invention to produce high quality recovered models even when the variations in sound speed exceed in fifty per cent of that of the typical soft tissue. Given that different tissues present different acoustic properties, the recovered models of acoustic properties can then be used as diagnostic tools.

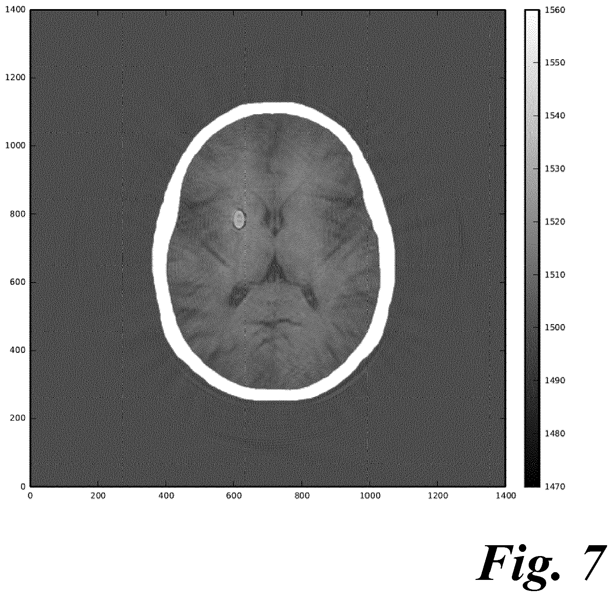

Figure 7 shows the results of the use of FWI to recover the target model from the starting model of Figure 6. Figure 7 is the result of running 100 iterations of FWI, increasing the frequency content in the data from 400 kHz up to 1.3 MHz. As shown, both the general brain structure and the clot are successfully recovered by FWI. In addition, the result suggests that the resolution of ultrasound images using waveform transmitted energy could match that of MRI images. Traditional ultrasound images tend to be of much lower resolution because they only exploit the information contained in the reflections.

Consequently, the method of the present invention has the ability to resolve soft tissue in situations where presence of bone and/or gas would prevent other ultrasound methods from obtaining images of sufficient resolution or detail to be useful in a diagnostic context.

Whilst the above example has been illustrated in relation to an ultrasound scan of the head (i.e. skull and brain), this is not to be taken as limiting. The present invention has applicability to regions of the body which traditionally cannot be imaged using conventional ultrasound methods. In other words, the present invention can be used to image areas of the body containing bone and/or gas which would cause significant difficulties for conventional ultrasound imaging.

The present invention is of application in situations where body tissues to be imaged are formed from tissue which has a speed of sound in said material which lies outside the range of 700 to 2300 m/s. The bones of the skull, bones in general, air, gas, metal, and most medical implants lie outside this 50% range of 700 to 2300 m/s with respect to the speed of sound in soft tissue. Note that soft tissue has a speed of sound therein within a few percent of 1500 m/s (around 1450 m/s for fat to about 1730 m/s for skin), whereas air has an approximate acoustic velocity of 350 m/s and bone 3000 m/s.

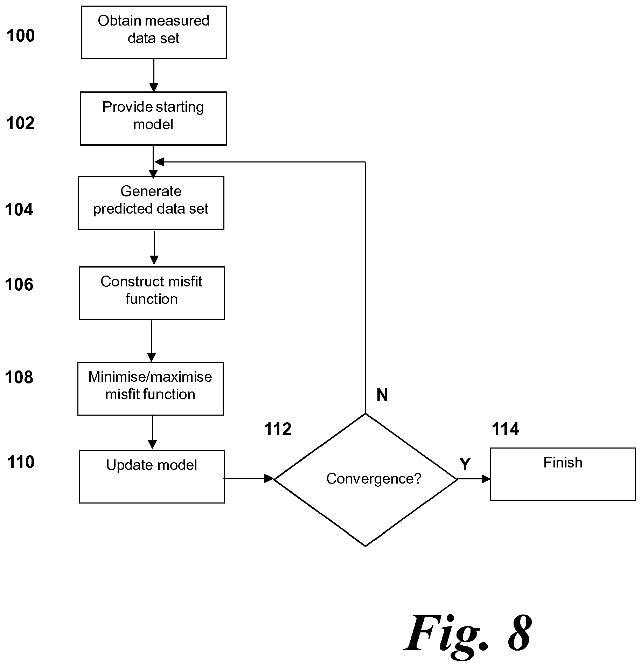

A generalised method of an embodiment of the invention will now be described with reference to Figure 8. The embodiment follows the described procedure as set out above.

*Step 100: Obtain observed data set*

Initially, it is necessary to obtain a set of experimentally gathered data in order to initiate the imaging procedure. This may be gathered by an experimental arrangement 10 such as the set up shown and described with reference to Figures 1 and 2.

Any suitable region of the body of a subject may be imaged using the experimental arrangement. Whilst the example above illustrates the head of a subject being imaged, this need not be the case. The present invention can be used to image areas of the body containing bone and/or gas which would cause significant difficulties for conventional ultrasound imaging, for example, the stomach, arms, legs, liver, chest and other regions not comprised solely of substantially homogeneous soft tissue.

The imaging may be done with any configuration of sensor. If the arrangement of Figures 1 and 2 is used, then the imaging may include a plurality of different measurements taken at different angles A to the horizontal.

The gathered ultrasonic data may be optionally pre-processed in various ways including by propagating numerically to regions of the model where experimental data have not been acquired directly. A person skilled in the art would be able to design and undertake such pre-processing as might be necessary or desirable. After such pre-processing, the resultant experimentally gathered ultrasonic data set is known as an "observed ultrasonic data set".

The observed ultrasonic data set may comprise multiple source 14 emissions. The data comprises pressure as a function of receiver position (on the x-axis) with respect to time (on the y-axis).

The trace data comprises a plurality of observed data points. Each measured discrete data point has a minimum of seven associated location values - three spatial dimensions (*x*, *y* and *z*) for receiver (or detector) position (*r*), three spatial dimensions (*x*, *y*, *z*) for source location (*s*), and one temporal dimension measuring the time of observation relative to the time of source initiation, together with pressure magnitude data. The seven coordinates for each discrete data point define its location in space and time. It also comprises one or more measurement parameters which denote the physical property being measured. In this embodiment, a single measurement parameter, pressure is measured. The observed data set is defined as $d_{obs}(\textbf{\textit{r}},\textbf{\textit{s}},t)$ and, in this embodiment, is in the time domain. For clarity, the following discussion considers a single source-receiver pair and so **r, s** are not needed.

The actual gathering of the ultrasonic data set is described here for clarity. However, this is not to be taken as limiting and the gathering of the data may or may not form part of the present invention. The present invention simply requires a real-world observed ultrasonic data set upon which analysis can be performed to facilitate medical imaging of a bone- or gas-containing region of the body of a subject.

The method now proceeds to step 102.

*Step 102: Provide starting model*

At step 102, an initial starting model of the region of the body to be imaged is provided. The model may be provided in either a two dimensional or a three dimensional form. Whilst the illustrated examples are of two dimensional form, the skilled person would be readily aware that the present invention is applicable to three dimensional approaches.

The generated model consists of values of the coefficient $V_p$ and, possibly, other physical values or coefficients, typically defined over a discrete grid representing the body region to be imaged.

The accuracy of the starting model is of significant importance to successfully image regions of the body that include bone and/or gas. It is of particular significance with regard to imaging of the head since the skull is a closed system and the measurements depend heavily upon the properties of the skull. Therefore, a starting model with an effective starting configuration for the skull is critical for successful convergence.

Therefore, it is necessary to ensure that the starting model is sufficiently representative of the region of the skull through which imaging is to take place in order to ensure successful imaging of the brain and soft tissue within.

The step of providing a starting model that is representative of the skull involves the selection, generation and/or modification of a starting model for the skull for use with the imaging method. These alternatives will now be described.

The skull component of the starting model may be generated in a number of different, non-limiting, ways. However, they can broadly be placed in two categories. The first is where one or more starting model skull components are generated in advance of the imaging procedure and form a database of starting model skull components. An appropriate skull component is then selected based on an empirical parameter relating to the subject or a measurement carried out on the subject.

The second is that the skull component of the starting model is procedurally generated or modified in response to an empirical parameter or measurement of the subject.

Firstly, it is necessary to acquire data in advance of the imaging measurement in order to generate one or more starting models. Key to the process is an accurate starting model of the skull.

This may be done in numerous ways. For example, reflection ultrasound methods could be used to obtain a starting model for the skull. This may involve, for example, using high frequencies or focused beams.

Alternatively or additionally, low intensity X-ray computed tomography (CT) scans could be used to provide an estimation of the skull properties which can be converted to velocities to generate a starting model. This data can be acquired beforehand either on the subject (e.g. in a screening process) or from a range of different subjects. This method can be done for bone or in the presence of air.

Alternatively, data may be obtained from other sources (e.g. MRI). As a further alternative, low-frequency transmission and reflection ultrasound could be used with FWI to obtain a full model of the skull. The low frequencies mean that cycle skipping can be avoided to build a better skull component of the starting model which is closer to the global minimum.

However, the method of acquisition of the starting model skull component is not material to the present invention. What is of benefit is that one or more skull components of the starting model are available to be selected from or modified in response to empirical data or measurements of the subject to be imaged.

The next stage is to acquire data relating to the subject to be imaged. This may be done in any suitable manner. The data acquired may relate to physical measurements made on the subject in situ, general characterising empirical data such as age, sex, weight, height or a combination of the above.

If measurements are made on the subject, this is to determine the structure and morphology of at least the skull. Key data to be acquired may relate to the thickness, composition and morphology.

The measurements made on the subject may include the observed data set acquired in step 100 as will be described below. Alternatively or additionally, other measurements may be used. For example, shear sensors could be placed on the skull and the skull excited with a shear stress. The surface waves propagating along the skull could then be measured and used to obtain information about the thickness and morphology of the skull.

Alternatively or additionally, lasers and/or direct physical measurements could be used to measure the outer geometry of the head.

As a further alternative or addition, the observed data set acquired in step 100 could be used to inform the choice of starting model. For example, in the case that a database of predetermined starting models has been provided, a corresponding predicted data set for each starting model could also be provided. Then, at least a part of the observed data set acquired in step 100 could be matched with the starting model predicted data sets to find the closest match. The starting model which represents the closest match could then be used in the full imaging process.

As a further alternative or addition, a part of the observed data set, for example at early travel times (representing reflections or refractions from the skull), could be used to generate or modify a model of the skull to generate a more accurate starting model for the full inversion process.

Any of the above empirical data or measured data parameters could be used to select from a pre-determined group of starting model skull components or to modify or generate a starting model skull component which has suitable accuracy to enable the imaging method to be carried out.

Once the starting model skull component has been generated, then a soft tissue component can be added to form the full starting model for the method of the present invention.

Since, as described above, the accuracy of the soft tissue component of the starting model is less critical than that of the skull component, the soft tissue component may comprise simply a homogeneous layer having an acoustic velocity around 1500 m/s (which is typical of soft tissue). Alternatively, other configurations of soft tissue component may be provided to account for the acoustic velocity of soft tissue varying from around 1450 m/s for fat to about 1730 m/s for skin. However, it is generally not necessary to provide for the variations in morphology and structure as is required from the skull component.

In summary, then, the starting model comprises a first component including elements having an acoustic velocity in excess of 2300 m/s (in the case of a skull), and a second component comprising elements having an acoustic velocity within the range of 1400 - 1750 m/s. This is the case for a starting model of the head as described in this non-limiting embodiment. However, other values may be used for the two components if other body parts are to be imaged (described later)

The method then proceeds to step 104.

*Step 104: Generate predicted data set*

At step 104, a predicted ultrasonic data set is generated. The predicted data is required to correspond to the same source-receiver location data positions as the actual measured trace data from the ultrasonic measurement so that the modelled and observed data can be compared. In other words, the predicted data set corresponds discrete point to discrete point to the observed data set. The predicted data set is generated for the same measurement parameter(s) at the same frequency or frequencies.

Predicted ultrasonic data may be generated based on an analysis of the acoustic isotropic two-way wave equation as set out below in equation 17):

$$17) \qquad \frac{1}{c^2}\frac{\partial^2 p}{\partial t^2} - \rho\nabla.\left(\frac{1}{\rho}\nabla p\right) = s$$

where the acoustic pressure *p* and driving source s vary in both space and time, and the acoustic velocity c and density ρ vary in space. This equation applies to small-amplitude pressure waves propagating within an inhomogeneous, isotropic, non-attenuating, non-dispersive, stationary, fluid medium. It is relatively straightforward to add elastic effects, attenuation and anisotropy to the wave equation. Introduction of these parameters changes the detailed equations and numerical complexity, but not the general approach.

The wave equation 17) represents a linear relationship between a wave field **p** and the source **s** that generates the wave field. After discretisation (with, for example, finite differences) we can therefore write equation 17) as a matrix equation 18):

$$18) \qquad\qquad\qquad \textbf{Ap = s}$$

where **p** and **s** are column vectors that represent the source and wavefield at discrete points in space and time, and **A** is a matrix that represents the discrete numerical implementation of the operator set out in equation 3):

$$19) \qquad \frac{1}{c^2}\frac{\partial^2}{\partial t^2} - \rho\nabla.\left(\frac{1}{\rho}\nabla\right)$$

Although the wave equation represents a linear relationship between **p** and **s**, it also represents a non-linear relationship between a model **m** and wavefield **p**. Thus equation 17) can be rewritten as equation 20):

$$20) \qquad\qquad\qquad \textbf{G(m) = p}$$

where **m** is a column vector that contains the model parameters. Commonly these will be
the values of *c* (and ρ if density is an independent parameter) at every point in the model, but they may be any set of parameters that is sufficient to describe the model, for example slowness 1/*c*, acoustic modulus $c^2 p$, or impedance *cp*.

In equation 20), **G** is not a matrix. Instead it is a non-linear Green's function that describes how to calculate a wavefield **p** given a model **m**.

From the above analysis, predicted ultrasonic data can be generated for one or more physical parameters in the time domain. If done in the frequency domain it could be done for one or more selected frequencies. This forms the predicted ultrasonic data set $d_{pred}$. The method now proceeds to step 106.

*Step 106: Construct misfit function*

At step 106, a misfit function is configured. In one example, the misfit function (or objective function) is configured to measure the dis-similarity between the observed and predicted data sets. Alternatively, an objective function may be configured that measures similarity; in this case, step 108 will operable to maximise rather than minimise the objective function.

The method proceeds to step 108.

*Step 108: Minimise or maximise the misfit function*

This may be done by any suitable method. In this embodiment, the gradient method is used to minimise the misfit between the two data sets.

The method then proceeds to step 110.

*Step 110: Update model*

At step 110, the model is updated using the gradient obtained in step 108. The model update derives from the gradient of equation 11) i.e. the partial derivative with respect to a point perturbation of the model m at each position. Ultimately gradients from separate sources will summed when forming the final model update.

As with the computational structure of conventional FWI method as used in seismic modelling, this is the product of two wavefields: an incident wavefield emitted by a source at a source location and a back-propagated wavefield which is emitted by a (multi-point) source located at the receiver positions.

As noted above, gradient methods can be enhanced using an approximate form for the Hessian and conjugate directions for the model update. Further a step-length is then calculated to scale the search direction and give the final model update.

For any useful measure of misfit or similarity the predicted ultrasonic data set will move towards the observed ultrasonic data set. Thus, the starting model will move towards the true model. The method now proceeds to step 112.

*Step 112: Convergence criteria met?*

At step 112 it is determined whether convergence criteria have been met. For example, the method may be deemed to have reached convergence when the difference between the data sets reaches a threshold percentage or other value. If the criteria as set out above have been met, then the method proceeds to step 114 and finishes with the resultant model generated. If the criteria have not been met, then the method proceeds back to repeat steps 104 to 110 as discussed above.

*Step 114: Finish*

When, at step 114, it has been determined that the convergence criteria has been met, the method finishes and the modelled region of the subject's body is deemed to be sufficiently accurate to be used for medical imaging.

Information can then be extracted from the imaged region of the subject's body. For example, the imaged region may be used to identify a pathology or to assist in the diagnosis of a pathology.

If applied to the head, the imaging method may be used to detect the presence and/or to determine the absence of congenital and/or acquired abnormalities in tissue composition and/or morphology within a region or regions within the intra-cranial cavity.

The images may also be used, for example to detect, identify, characterise, locate and/or differentiate between normal tissue and abnormal tissue caused by intra-cranial pathologies of vascular supply to the brain involving an interruption to, and/or a reduction of, blood flow to brain tissue and/or extradural, subdural, subarachnoid, intra-cerebral and/or intra-ventricular haemorrhage.

Alternatively, if the imaged region of the body is one containing bones then the imaging method could be used to detect, identify, characterise and/or locate any abnormality due to muscular or osseus injuries.

If the torso is the subject of the imaging, the method could be used to identify the totality of the viscera to detect, identify, characterise and/or locate any abnormality, for instance, due to a tumour in the pancreas or kidney stones.

If a pregnant woman is imaged, the recovered acoustic properties of the abdominal part of a pregnant woman to detect, identify, characterise, locate and/or image a foetus.

Alternatively, the method can be used to detect, identify, characterise and locate tumours that are otherwise obscured by the presence of bone and/or gas within the region being imaged.

Whilst the embodiment of the method above has been described with specific references to the head and skull, the method is applicable to other regions of the body which may contain interfaces between bone, gas and/or soft tissue and which cannot be imaged by conventional means.

The described method of the present invention can be generalised to other parts of the body where a starting model is built up and comprises at least two components-a first component comprising a plurality of model parameters representative of the physical properties and morphology of bone and/or gas within the body part of the subject to be imaged and having at least one modelled region having an acoustic velocity below 700 m/s and/or above 2300 m/s, and a second component comprising a plurality of parameters representative of the physical properties of the soft tissue within the body part of the subject to be imaged. In general, the soft tissue component of the starting model comprises elements having an acoustic velocity within the range of 1400 - 1750 m/s.

In some cases, a variation to the described embodiment of the method may be required. For example, the method of obtaining a starting model may be different for certain regions of the body when compared to the head. It may, for instance, not be possible to maintain a database of different starting model components in cases where there may be large physical, genetic and/or geometric differences between subjects.

For example, the imaging of a joint may be problematic since it is challenging to provide a range of starting models which capture the possible variations in joint angle, size, construction etc. However, imaging of such regions is generally not as time-sensitive as imaging of the head and brain (e.g. in the case of stroke). Therefore, a measurement of the structure of the body region may be made simultaneously or sequentially using, for example, a low intensity X-ray CT image from which a starting model is subsequently derived.

The skilled person would be readily aware of the suitable environments in which data could be gathered for imaging and analysis purposes as set out in the present disclosure.

In aspects, the embodiments described herein relate to a method of extracting information from a digital image. However, the embodiments described herein are equally applicable as an instruction set for a computer for carrying out said method or as a suitably programmed computer.

The methods described herein are, in use, executed on a suitable computer system or device running one or more computer programs formed in software and/or hardware and operable to execute the above method. A suitable computer system will generally comprise hardware and an operating system.

The term 'computer program' is taken to mean any of (but not necessarily limited to) an application program, middleware, an operating system, firmware or device drivers or any other medium supporting executable program code.

The term 'hardware' may be taken to mean any one or more of the collection of physical elements that constitutes a computer system/device such as, but not limited to, a processor, memory device, communication ports, input/output devices. The term 'firmware' may be taken to mean any persistent memory and the program code/data stored within it, such as but not limited to, an embedded system. The term 'operating system' may taken to mean the one or more pieces, often a collection, of software that manages computer hardware and provides common services for computer programs.

The comparison step may also be conducted making use of previous measurements on a healthy or diseased population of reference joints for which values or average values are stored in a database or memory location in such a computer. The computer may be programmed to display the results of the comparison as a read out.

The methods described herein may be embodied in one or more pieces of software and/or hardware. The software is preferably held or otherwise encoded upon a memory device such as, but not limited to, any one or more of, a hard disk drive, RAM, ROM, solid state memory or other suitable memory device or component configured to software. The methods may be realised by executing/running the software. Additionally or alternatively, the methods may be hardware encoded.

The method encoded in software or hardware is preferably executed using one or more processors. The memory and/or hardware and/or processors are preferably comprised as, at least part of, one or more servers and/or other suitable computing systems.

**Claims**

1. A non-invasive method of generating image data of intra-cranial tissue using ultrasound energy that is transmitted across a head of a subject through the skull of the subject, the method comprising the steps of:

   a) providing (100) an ultrasound observed data set derived from a measurement of one or more ultrasound waveforms generated by at least one source of ultrasound energy, the ultrasound energy being detected by a plurality of receivers located at an opposing side of a region within the intra-cranial cavity with respect to at least one source such that the receivers detect ultrasound waveforms from the source which have been transmitted through the skull and intra-cranial cavity, the observed data set comprising a plurality of observed data values;
   b) providing (102) at least one starting model for at least a portion of the head comprising a skull component and a soft tissue component, the skull component comprising a plurality of model parameters representative of the physical properties and morphology of the skull through which intra-cranial tissue is being imaged, and the soft tissue component comprising a plurality of parameters representative of the physical properties of the intra-cranial tissue being imaged;
   c) generating (104) a predicted data set comprising a plurality of predicted data values from the starting model of the skull and of the intra-cranial tissue;
   d) comparing the observed and predicted data values in order to generate an updated model of at least one physical property within at least a region of the intra-cranial cavity; and
   e) using the updated model to image a region of the inter-cranial cavity to identify tissue composition and/or morphology within the intra-cranial cavity.

2. A method according to claim 1, wherein step b) comprises:
f) acquiring subject data relating to the subject, and providing at least the skull component of the starting model based on the acquired subject data, wherein the acquired subject data is obtained from a measurement performed on the subject and/or from empirical data relating to the subject.

3. A method according to claim 2, wherein the skull component is selected from a group of predetermined skull components based at least in part upon a matching process between at least a part of the observed data set and a group of starting predicted data sets generated from the respective group of the skull components of the starting models.

4. A method according to any one of the preceding claims, wherein the ultrasound data set is derived from a measurement of ultrasound waveforms generated by a plurality of sources of ultrasound energy, the ultrasound energy being detected by a plurality of receivers, wherein the sources and receivers are located such that the receivers detect transmitted ultrasound waveforms from the sources which have been transmitted through the skull and inter-cranial cavity and/or reflected ultrasound waveforms that have been reflected by the inner and/or outer boundaries of the skull.

5. A method according to claim 4, wherein at least the reflected waveforms of the observed data set are used to recover a numerical model of the geometry of at least a part of the skull, at least a part of the skull component of the starting model provided in step b) being derived from the numerical model, wherein, analysing at least the transmitted waveforms of the said observed dataset in order to recover a numerical model of at least one physical property within at least a region of the intra-cranial cavity, and analysing both reflected and transmitted waveforms in order to recover at least one physical property of the skull, are performed by comparison of the observed reflected and transmitted waveforms with predicted waveforms that have been simulated numerically and/or generated experimental using at least one numerical and/or physical and/or in vivo predicted model for which the relevant geometry and property or properties are known and/or can be inferred or approximated.

6. A non-invasive method of generating image data of a body part of a subject using ultrasound energy that is transmitted through the body part of the subject, the body part containing at least one interface between bone, soft tissue and/or gas, the method comprising the steps of:

a) providing (100) an ultrasound observed data set derived from a measurement of one or more ultrasound waveforms generated by at least one source of ultrasound energy, the ultrasound energy being detected by a plurality of receivers located at an opposing side of a region within the body part with respect to at least one source such that the receivers detect ultrasound waveforms from the source which have been transmitted through the body part, the observed data set comprising a plurality of observed data values;
b) providing (102) at least one starting model representative of the body part being imaged, the starting model comprising first and second components, the first component comprising a plurality of model parameters representative of the physical properties and morphology of the bone and/or gas within the body part of the subject to be imaged and having at least one modelled region having an acoustic velocity below 700 m/s and/or above 2300 m/s and the second component comprising a plurality of parameters representative of the physical properties of the soft tissue within the body part of the subject to be imaged;
c) generating (104) a predicted data set comprising a plurality of predicted data values from the starting model;
d) comparing the observed and predicted data values in order to generate an updated model of at least one physical property within at least a region of the body part; and
e) using the updated model to image a region of the body part to identify tissue composition and/or morphology within the body part.

7. A method according to claim 6, wherein step b) comprises:
f) acquiring subject data relating to the subject, and providing at least the first component of the starting model based on the acquired subject data, wherein the acquired subject data is obtained from a measurement performed on the subject and/or from empirical data relating to the subject.

8. A method according to claim 7, wherein the first component is selected from a group of predetermined components based on the acquired subject data, wherein the first component is selected from a group of predetermined first components based at least in part upon a matching process between at least a part of the observed data set and a group of starting predicted data sets generated from the respective group of the first components of the starting models.

9. A method according to any one of the preceding claims, wherein the ultrasound data set is derived from a measurement of ultrasound waveforms generated by a plurality of sources of ultrasound energy, the ultrasound energy being detected by a plurality of receivers, wherein the sources and receivers are located such that the receivers detect transmitted ultrasound waveforms from the sources which have been transmitted through the body part and/or reflected ultrasound waveforms that have been reflected by any inner and/or outer boundaries of the body part.

10. A method according to claim 9, wherein at least the reflected waveforms of the observed data set are used to recover a numerical model of the geometry of at least a part of the body part, at least a part of the first component of the starting model provided in step b) being derived from the numerical model, wherein, analysing at least the transmitted waveforms of the said observed dataset in order to recover a numerical model of at least one physical property within at least a region of the body part, and analysing both reflected and transmitted waveforms in order to recover at least one physical property of the body part, by comparison of the observed reflected and transmitted waveforms with predicted waveforms that have been simulated numerically and/or generated experimental using at least one numerical and/or physical and/or in vivo predicted model for which the relevant geometry and property or properties are known and/or can be inferred or approximated.

11. A method according to any of the preceding claims, wherein at least a portion of the said observed and predicted waveforms differ in phase by more than half a cycle at the lowest frequency present in the said observed dataset.

12. A method according to any one of the preceding claims, wherein step d) is performed using full waveform inversion analysis.

13. A computer system comprising a processing device configured to perform the method of any one of the preceding claims.

14. A computer readable medium comprising instructions configured when executed to perform the method of any one of claims 1 to 12.

15. A computer system comprising: a processing device, a storage device and a computer readable medium according to claim 14.

**Patentansprüche**

1. Nicht-invasives Verfahren zum Erzeugen von Bilddaten von intrakraniellem Gewebe unter Verwendung von Ultraschallenergie, die über einen Kopf eines Subjekts durch den Schädel des Subjekts übertragen wird, wobei das Verfahren die folgenden Schritte umfasst:

a) Bereitstellen (100) eines beobachteten Ultraschalldatensatzes, der aus einer Messung einer oder mehrerer Ultraschallwellenformen abgeleitet wird, die von mindestens einer Ultraschallenergiequelle erzeugt werden, wobei die Ultraschallenergie von einer Vielzahl von Empfängern detektiert wird, die sich auf einer gegenüberliegenden Seite eines Bereichs innerhalb der intrakraniellen Höhle mit Bezug auf mindestens eine Quelle befinden, sodass die Empfänger Ultraschallwellenformen von der Quelle detektieren, die durch den Schädel und die intrakranielle Höhle übertragen wurden, wobei der beobachtete Datensatz eine Vielzahl von beobachteten Datenwerten umfasst;
b) Bereitstellen (102) mindestens eines Ausgangsmodells für mindestens einen Teil des Kopfes, der eine Schädelkomponente und eine Weichgewebekomponente umfasst, wobei die Schädelkomponente eine Vielzahl von Modellparametern umfasst, die für die physikalischen Eigenschaften und die Morphologie des Schädels repräsentativ sind, durch den intrakranielles Gewebe abgebildet wird, und die Weichgewebekomponente eine Vielzahl von Parametern umfasst, die für die physikalischen Eigenschaften des abgebildeten intrakraniellen Gewebes repräsentativ sind;
c) Erzeugen (104) eines vorhergesagten Datensatzes, der eine Vielzahl von vorhergesagten Datenwerten aus dem Ausgangsmodell des Schädels und des intrakraniellen Gewebes umfasst;
d) Vergleichen der beobachteten und der vorhergesagten Datenwerte, um ein aktualisiertes Modell von mindestens einer physikalischen Eigenschaft innerhalb mindestens eines Bereichs der intrakraniellen Höhle zu erzeugen; und
e) Verwenden des aktualisierten Modells zum Abbilden eines Bereichs der interkraniellen Höhle, um eine Gewebezusammensetzung und/oder Morphologie innerhalb der intrakraniellen Höhle zu identifizieren.

2. Verfahren nach Anspruch 1, wobei Schritt b) umfasst:
f) Erfassen von Subjektdaten, die sich auf das Subjekt beziehen, und Bereitstellen mindestens der Schädelkomponente des Ausgangsmodells basierend auf den erfassten Subjektdaten, wobei die erfassten Subjektdaten aus einer Messung, die an dem Subjekt durchgeführt wurde, und/oder aus empirischen Daten, die sich auf das Subjekt beziehen, erhalten werden.

3. Verfahren nach Anspruch 2, wobei die Schädelkomponente aus einer Gruppe von vorbestimmten Schädelkomponenten zumindest teilweise basierend auf einem Übereinstimmungsprozess zwischen mindestens einem Teil des beobachteten Datensatzes und einer Gruppe von vorhergesagten Ausgangsdatensätzen, die aus der jeweiligen Gruppe der Schädelkomponenten der Ausgangsmodelle erzeugt werden, ausgewählt wird.

4. Verfahren nach einem der vorhergehenden Ansprüche, wobei der Ultraschalldatensatz aus einer Messung von Ultraschallwellenformen abgeleitet wird, die durch eine Vielzahl von Ultraschallenergiequellen erzeugt werden, wobei die Ultraschallenergie durch eine Vielzahl von Empfängern detektiert wird, wobei die Quellen und Empfänger derart angeordnet sind, dass die Empfänger übertragene Ultraschallwellenformen von den Quellen, die durch den Schädel und die interkranielle Höhle übertragen wurden, und/oder reflektierte Ultraschallwellenformen, die von den inneren und/oder äußeren Grenzen des Schädels reflektiert wurden, detektieren.

5. Verfahren nach Anspruch 4, wobei mindestens die reflektierten Wellenformen des beobachteten Datensatzes verwendet werden, um ein numerisches Modell der Geometrie von mindestens einem Teil des Schädels zu gewinnen, wobei mindestens ein Teil der Schädelkomponente des in Schritt b) bereitgestellten Ausgangsmodells aus dem numerischen Modell abgeleitet wird, wobei ein Analysieren mindestens der übertragenen Wellenformen des beobachteten Datensatzes, um ein numerisches Modell von mindestens einer physikalischen Eigenschaft innerhalb mindestens eines Bereichs der intrakraniellen Höhle zu gewinnen, und ein Analysieren sowohl reflektierter als auch übertragener Wellenformen, um mindestens eine physikalische Eigenschaft des Schädels zu gewinnen, durch einen Vergleich der beobachteten reflektierten und übertragenen Wellenformen mit vorhergesagten Wellenformen durchgeführt werden, die numerisch simuliert und/oder experimentell unter Verwendung mindestens eines numerischen und/oder physikalischen und/oder in vivo vorhergesagten Modells, für das die relevante Geometrie und Eigenschaft oder Eigenschaften bekannt sind und/oder abgeleitet oder angenähert werden können, erzeugt wurden.

6. Nicht-invasives Verfahren zum Erzeugen von Bilddaten eines Körperteils eines Subjekts unter Verwendung von Ultraschallenergie, die durch den Körperteil des Subjekts übertragen wird, wobei der Körperteil mindestens eine Grenzfläche zwischen Knochen, Weichgewebe und/oder Gas enthält, wobei das Verfahren die folgenden Schritte umfasst:

a) Bereitstellen (100) eines beobachteten Ultraschalldatensatzes, der aus einer Messung einer oder mehrerer Ultraschallwellenformen abgeleitet wird, die von mindestens einer Ultraschallenergiequelle erzeugt werden, wobei die Ultraschallenergie von einer Vielzahl von Empfängern detektiert wird, die sich auf einer gegenüberliegenden Seite eines Bereichs innerhalb des Körperteils mit Bezug auf mindestens eine Quelle befindet, sodass die Empfänger Ultraschallwellenformen von der Quelle detektieren, die durch den Körperteil übertragen wurden, wobei der beobachtete Datensatz eine Vielzahl von beobachteten Datenwerten umfasst;
b) Bereitstellen (102) mindestens eines Ausgangsmodells, das für den abgebildeten Körperteil repräsentativ ist, wobei das Ausgangsmodell eine erste und eine zweite Komponente umfasst, wobei die erste Komponente eine Vielzahl von Modellparametern umfasst, die für die physikalischen Eigenschaften und die Morphologie des Knochens und/oder Gases innerhalb des Körperteils des abzubildenden Subjekts repräsentativ sind und mindestens einen modellierten Bereich aufweist, der eine Schallgeschwindigkeit unter 700 m/s und/oder über 2300 m/s aufweist, und die zweite Komponente eine Vielzahl von Parametern umfasst, die für die physikalischen Eigenschaften des Weichgewebes innerhalb des Körperteils des abzubildenden Subjekts repräsentativ sind;
c) Erzeugen (104) eines vorhergesagten Datensatzes, der eine Vielzahl von vorhergesagten Datenwerten aus dem Ausgangsmodell umfasst;
d) Vergleichen der beobachteten und vorhergesagten Datenwerte, um ein aktualisiertes Modell von mindestens einer physikalischen Eigenschaft innerhalb mindestens eines Bereichs des Körperteils zu erzeugen; und
e) Verwenden des aktualisierten Modells zum Abbilden eines Bereichs des Körperteils, um eine Gewebezusammensetzung und/oder Morphologie innerhalb des Körperteils zu identifizieren.

**7.** Verfahren nach Anspruch 6, wobei Schritt b) umfasst:
f) Erfassen von Subjektdaten, die sich auf das Subjekt beziehen, und Bereitstellen mindestens der ersten Komponente des Ausgangsmodells basierend auf den erfassten Subjektdaten, wobei die erfassten Subjektdaten aus einer Messung, die an dem Subjekt durchgeführt wurde, und/oder aus empirischen Daten, die sich auf das Subjekt beziehen, erhalten werden.

**8.** Verfahren nach Anspruch 7, wobei die erste Komponente aus einer Gruppe vorbestimmter Komponenten basierend auf den erfassten Subjektdaten ausgewählt wird, wobei die erste Komponente aus einer Gruppe von vorbestimmten ersten Komponenten zumindest teilweise basierend auf einem Übereinstimmungsprozess zwischen mindestens einem Teil des beobachteten Datensatzes und einer Gruppe von vorhergesagten Ausgangsdatensätzen, die aus der jeweiligen Gruppe der ersten Komponenten der Ausgangsmodelle erzeugt werden, ausgewählt wird.

**9.** Verfahren nach einem der vorhergehenden Ansprüche, wobei der Ultraschalldatensatz aus einer Messung von Ultraschallwellenformen abgeleitet wird, die durch eine Vielzahl von Ultraschallenergiequellen erzeugt werden, wobei die Ultraschallenergie durch eine Vielzahl von Empfängern detektiert wird, wobei die Quellen und Empfänger derart angeordnet sind, dass die Empfänger übertragene Ultraschallwellenformen von den Quellen, die durch den Körperteil übertragen wurden, und/oder reflektierte Ultraschallwellenformen, die von inneren und/oder äußeren Grenzen des Körperteils reflektiert wurden, detektieren.

**10.** Verfahren nach Anspruch 9, wobei mindestens die reflektierten Wellenformen des beobachteten Datensatzes verwendet werden, um ein numerisches Modell der Geometrie von mindestens einem Teil des Körperteils zu gewinnen, wobei mindestens ein Teil der ersten Komponente des in Schritt b) bereitgestellten Ausgangsmodells aus dem numerischen Modell abgeleitet wird, wobei ein Analysieren mindestens der übertragenen Wellenformen des beobachteten Datensatzes, um ein numerisches Modell von mindestens einer physikalischen Eigenschaft innerhalb mindestens eines Bereichs des Körperteils zu gewinnen, und ein Analysieren sowohl reflektierter als auch übertragener Wellenformen, um mindestens eine physikalische Eigenschaft des Körperteils zu gewinnen, durch einen Vergleich der beobachteten reflektierten und übertragenen Wellenformen mit vorhergesagten Wellenformen durchgeführt werden, die numerisch simuliert und/oder experimentell unter Verwendung mindestens eines numerischen und/oder physikalischen und/oder in vivo vorhergesagten Modells, für das die relevante Geometrie und Eigenschaft oder Eigenschaften bekannt sind und/oder abgeleitet oder angenähert werden können, erzeugt wurden.

**11.** Verfahren nach einem der vorhergehenden Ansprüche, wobei sich mindestens ein Teil der beobachteten und vorhergesagten Wellenformen in der Phase um mehr als einen halben Zyklus bei der niedrigsten in dem beobachteten Datensatz vorhandenen Frequenz unterscheidet.

**12.** Verfahren nach einem der vorhergehenden Ansprüche, wobei Schritt d) unter Verwendung einer vollständigen Wellenforminversionsanalyse durchgeführt wird.

**13.** Computersystem, umfassend eine Verarbeitungsvorrichtung, die dazu konfiguriert ist, das Verfahren nach einem der vorhergehenden Ansprüche durchzuführen.

**14.** Computerlesbares Medium, umfassend Anweisungen, die dazu konfiguriert sind, wenn sie ausgeführt werden, das Verfahren nach einem der Ansprüche 1 bis 12 durchzuführen.

**15.** Computersystem, umfassend: eine Verarbeitungsvorrichtung, eine Speichervorrichtung und ein computerlesbares Medium nach Anspruch 14.

**Revendications**

**1.** Procédé non invasif de génération de données d'image de tissu intracrânien à l'aide d'une énergie ultrasonore qui est transmise à travers une tête d'un sujet par le crâne du sujet, le procédé comprenant les étapes suivantes :

a) fournir (100) un ensemble de données ultrasonores observées dérivées d'une mesure d'une ou plusieurs formes d'ondes ultrasonores générées par au moins une source d'énergie ultrasonore, l'énergie ultrasonore étant détectée par une pluralité de récepteurs situés d'un côté opposé d'une région à l'intérieur de la cavité intracrânienne par rapport à au moins une source de sorte que les récepteurs détectent les formes d'ondes ultrasonores de la source qui ont été transmises à travers le crâne et la cavité intracrânienne, l'ensemble de données observées comprenant une pluralité de valeurs de données observées ;

b) fournir (102) au moins un modèle de départ d'au moins une partie de la tête comprenant un élément de crâne et un élément de tissu mou, l'élément de crâne comprenant une pluralité de paramètres du modèle représentatif des propriétés physiques et de la morphologie du crâne à travers lequel une image d'un tissu intracrânien est formée et l'élément de tissu mou comprenant une pluralité de paramètres représentatifs des propriétés physiques du tissu intracrânien dont l'image est formée ;

c) générer (104) un ensemble de données prédites comprenant une pluralité de valeurs de données prédites à partir du modèle de départ du crâne et du tissu intracrânien ;

d) comparer des valeurs de données observées et prédites afin de générer un modèle mis à jour d'au moins une propriété physique à l'intérieur d'au moins une région de la cavité intracrânienne ; et

e) utiliser le modèle mis à jour pour obtenir l'image d'une région de la cavité intercrânienne afin d'identifier la composition et/ou la morphologie du tissu à l'intérieur de la cavité intracrânienne.

2. Procédé selon la revendication 1, dans lequel étape b) comprend :

f) l'acquisition de données du sujet se rapportant au sujet et la fourniture d'au moins l'élément de crâne du modèle de départ sur la base des données acquises du sujet, dans lesquelles les données acquises du sujet sont obtenues à partir d'une mesure réalisée sur le sujet et/ou à partir de données empiriques se rapportant au sujet.

3. Procédé selon la revendication 2, dans lequel l'élément de crâne est sélectionné parmi un groupe d'éléments de crâne prédéfinis sur la base, au moins en partie, d'un processus de mise en correspondance entre au moins une partie de l'ensemble de données observées et un groupe d'ensembles de données de départ prédites générées à partir du groupe respectif d'éléments de crâne des modèles de départ.

4. Procédé selon une quelconque des revendications précédentes, dans lequel l'ensemble de données ultrasonores est dérivé d'une mesure des formes d'ondes générées par une pluralité de sources d'énergie ultrasonore, l'énergie ultrasonore étant détectée par une pluralité de récepteurs, dans lequel les sources et les récepteurs sont situés de sorte que les récepteurs détectent les formes d'ondes ultrasonores transmises à partir des sources qui ont été transmises à travers le crâne et la cavité intercrânienne et/ou les formes d'ondes ultrasonores réfléchies qui ont été réfléchies par les limites interne et/ou externe du crâne.

5. Procédé selon la revendication 4, dans lequel au moins les formes d'ondes réfléchies de l'ensemble de données observées sont utilisées pour récupérer un modèle numérique de la géométrie d'au moins une partie du crâne, au moins une partie de l'élément de crâne du modèle de départ fourni à l'étape b) étant dérivée du modèle numérique, dans lequel, l'analyse d'au moins les formes d'ondes transmises dudit ensemble de données observées afin de récupérer un modèle numérique d'au moins une propriété physique à l'intérieur d'au moins une région de la cavité intracrânienne et l'analyse à la fois des formes d'ondes réfléchies et transmises afin de récupérer au moins une propriété physique du crâne, sont réalisées par comparaison des formes d'ondes réfléchies et transmises observées avec les formes d'ondes prédites qui ont été simulées numériquement et/ou générées expérimentalement à l'aide d'au moins un modèle prédit numérique et/ou physique et/ou in vivo pour lequel la géométrie, et la ou les propriétés pertinentes sont connues et/ou peuvent être inférées ou approximées.

6. Procédé non invasif de génération de données d'image d'une partie du corps d'un sujet à l'aide d'une énergie ultrasonore qui est transmise à travers la partie du corps du sujet, la partie du corps contenant au moins une interface entre os, tissu mou et/ou gaz, le procédé comprenant les étapes suivantes :

a) fournir (100) un ensemble de données ultrasonores observées dérivées d'une mesure d'une ou plusieurs formes d'ondes ultrasonores générées par au moins une source d'énergie ultrasonore, l'énergie ultrasonore étant détectée par une pluralité de récepteurs situés d'un côté opposé d'une région à l'intérieur de la partie du corps par rapport à au moins une source, de sorte que les récepteurs détectent les formes d'ondes ultrasonores de la source qui ont été transmises à travers la partie du corps, l'ensemble de données observées comprenant une pluralité de valeurs de données observées ;

b) fournir (102) au moins un modèle de départ représentatif de la partie du corps dont l'image est formée, le modèle de départ comprenant des premier et second éléments, le premier élément comprenant une pluralité de paramètres de modèle représentatifs des propriétés physiques et de la morphologie de l'os et/ou du gaz à l'intérieur de la partie du corps du sujet dont l'image doit être formée et présentant au moins une région modélisée possédant une vitesse acoustique inférieure à 700 m/s et/ou supérieure à 2300 m/s, et le second élément comprenant une pluralité de paramètres représentatifs des propriétés physiques du tissu mou à l'intérieur de la partie du corps du sujet dont l'image doit être formée ;

c) générer (104) un ensemble de données prédites comprenant une pluralité de valeurs de données prédites à partir du modèle de départ ;

d) comparer des valeurs de données observées et prédites afin de générer un modèle mis à jour d'au moins une propriété physique à l'intérieur d'au moins une région de la partie du corps ; et

e) utiliser le modèle mis à jour pour former une image d'une région de la partie du corps afin d'identifier la composition et/ou la morphologie du tissu à l'intérieur de la partie du corps.

7. Procédé selon la revendication 6, dans lequel étape b) comprend :

f) l'acquisition de données du sujet se rapportant au sujet et la fourniture d'au moins le premier élément du modèle de départ sur la base des données de sujet acquises, dans lequel les données de sujet acquises sont obtenues à partir d'une mesure réalisée sur le sujet et/ou à partir de données empiriques se rapportant au sujet.

8. Procédé selon la revendication 7, dans lequel le premier élément est sélectionné parmi un groupe d'éléments prédéfinis sur la base des données de sujet acquises, dans lequel le premier élément est sélectionné parmi un groupe de premiers éléments prédéfinis sur la base, au moins en partie, d'un processus de mise en correspondance entre au moins une partie de l'ensemble de données observées et un groupe d'ensembles de données de départ prédites générées à partir du groupe respectif des premiers éléments des modèles de départ.

9. Procédé selon une quelconque des revendications précédentes, dans lequel l'ensemble des données ultrasonores est dérivé d'une mesure des formes d'ondes générées par une pluralité de sources d'énergie ultrasonore, l'énergie ultrasonore étant détectée par une pluralité de récepteurs, dans lequel les sources et les récepteurs sont situés de sorte que les récepteurs détectent les formes d'ondes ultrasonores transmises à partir des sources qui ont été transmises à travers la partie du corps, et/ou les formes d'ondes ultrasonores réfléchies qui ont été réfléchies par de quelconques limites internes et/ou externes de la partie du corps.

10. Procédé selon la revendication 9, dans lequel au moins les formes d'ondes réfléchies de l'ensemble de données observées sont utilisées pour récupérer un modèle numérique de la géométrie d'au moins une partie de la partie du corps, au moins une partie du premier élément du modèle de départ fourni à l'étape b) étant dérivée du modèle numérique, dans lequel l'analyse d'au moins les formes d'ondes transmises dudit ensemble de données observées afin de récupérer un modèle numérique d'au moins une propriété physique à l'intérieur d'au moins une région de la partie du corps et l'analyse à la fois des formes d'ondes réfléchies et transmises afin de récupérer au moins une propriété physique de la partie du corps sont effectuées par comparaison des formes d'ondes réfléchies et transmises observées avec les formes d'ondes prédites qui ont été simulées numériquement et/ou générées expérimentales à l'aide d'au moins un modèle numérique et/ou physique et/ou in vivo prédit pour lequel la géométrie et la ou les propriétés pertinentes sont connues et/ou peuvent être inférées ou approximées.

11. Procédé selon l'une quelconque des revendications précédentes, dans lequel au moins une partie desdites formes d'ondes observées et prédites diffèrent en phase de plus d'un demi-cycle à la fréquence la plus basse présente dans ledit ensemble de données observées.

12. Procédé selon l'une quelconque des revendications précédentes, dans lequel l'étape d) est réalisée à l'aide de l'analyse complète d'inversion des formes d'ondes.

13. Système informatique comprenant un dispositif de traitement configuré pour réaliser le procédé de l'une quelconque des revendications précédentes.

14. Support lisible par ordinateur comprenant des instructions configurées, lorsqu'elles sont exécutées pour réaliser le procédé selon l'une quelconque des revendications 1 à 12.

15. Système informatique comprenant : un dispositif de traitement, un dispositif de mémoire et un support lisible par ordinateur selon la revendication 14.

*Fig. 1*

*Fig. 2*

*Fig. 3*

*Fig. 4*

Fig. 5

Fig. 6

*Fig. 7*

**100** Obtain measured data set

**102** Provide starting model

**104** Generate predicted data set

**106** Construct misfit function

**108** Minimise/maximise misfit function

**110** Update model

**112** Convergence?

N

Y

**114** Finish

*Fig. 8*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8834376 B

- US 2012283566 A

**Non-patent literature cited in the description**

- **J YLITALO.** Ultrasound reflection mode computed tomography through a skullbone. *IEEE transactions on biomedical engineering,* November 1990, vol. 37 (11

- **K.A. DINES et al.** Computerized ultrasound tomography of the human head: Experimental results. *Ultrasonic Imaging,* 1981, vol. 3, 342-351